# EUROPÄISCHE PATENTSCHRIFT

(11) **EP 0 308 665 B1**
(45) Veröffentlichungstag und Bekanntmachung des Hinweises auf die Patenterteilung: **14.04.1993**
(21) Anmeldenummer: 88113698.0
(22) Anmeldetag: 21.12.1982
(51) Int. Cl.: C07C 229/64, C07C 229/28, C07C 215/08, C07C 279/32, A61K 9/00, A61K 47/00, A61K 31/615

(54) **Salze der 5-Aminosalicylsäure und sie enthaltende Arzneimittelzubereitungen**
5-Aminosalicylic acid salts and pharmaceutical preparations containing them
Sels de l'acide 5-aminosalicylique et préparations pharmaceutiques qui les contiennent

(30) Priorität: 23.12.1981 DE 3151196
(43) Veröffentlichungstag der Anmeldung: 29.03.1989
(62) Teilanmeldung aus: 82111850.2
(73) Patentinhaber: Bauer, Kurt H., Prof. Dr., D-79112 Freiburg (DE)
(72) Erfinder: Bauer, Kurt H., Prof. Dr., D-79112 Freiburg (DE)
(74) Vertreter: Kraus, Walter, Dr.

(56) Entgegenhaltungen:
- WO-A-81/02671
- GB-A- 2 059 768
- US-A- 3 069 321

## Beschreibung

Die Erfindung betrifft Salze der 5-Aminosalicylsäure und sie enthaltende Arzneimittelzubereitungen.

Die 5-Aminosalicylsäure wurde früher schon zusammen mit der 4-Aminosalicylsäure (PAS) als Tuberculostaticum geprüft, aber für deutlich weniger wirksam befunden. Die 4-Aminosalicylsäure ist auch, verglichen mit der 5-Aminosalicylsäure, wesentlich stabiler und kann deshalb im Gegensatz zu der 5-Aminosalicylsäure ohne Probleme als Natriumsalz appliziert werden.

Zur Behandlung der Colitis ulcerosa und des Morbus Crohn wurde bisher Salazosulfapyridin verwendet. Diese Substanz wird im Darm durch Bakterien oder Enzyme in 5-Aminosalicylsäure und in das Sulfonamid Sulfapyramidin gespalten. Es war lange Zeit unklar, welche der drei Substanzen das wirksame Agens darstellt. Neuere Untersuchungen haben gezeigt, daß die 5-Aminosalicylsäure der wirksame Bestandteil ist, während das Sulfapyramidin hauptsächlich für Nebenwirkungen verantwortlich zeichnet.

Mit der 5-Aminosalicylsäure wurden schon Therapieversuche mit Suppositorien und Einläufen gemacht. Die Anwendung von Suppositorien und insbesondere die von Einläufen ist jedoch unangenehm, insbesondere braucht man für die Verabreichung von Einläufen viel Zeit. Nicht erfolgreich waren bisher die Versuche mit Tabletten. Wegen der schlechten Löslichkeit und/oder der schlechten Stabilität blieb somit die praktische Anwendung der 5-Aminosalicylsäure bisher erfolglos. Aus diesen Gründen wurde die 5-Aminosalicylsäure in der Vergangenheit immer in Form des Salazosulfapyridins verwendet, und auch heute wird in der aktuellen Therapie immer noch Salazosulfapyridin eingesetzt. Man nimmt an, daß die 5-Aminosalicylsäure bei der Spaltung im Dickdarm in "statu nascendi" in feinstverteilter Form anfällt, d.h. in einer Dispersität, die mechanisch durch Mahlen nicht zu bewerkstelligen ist, und auf diese Weise wirksam wird.

Die 5-Aminosalicylsäure (5-Amino-2-hydroxy-benzoesäure) ist in den meistgebräuchlichen Lösungsmitteln nur sehr schwer oder nicht löslich. Es ist deshalb praktisch unmöglich, auf diesem Wege die Verbindung zur Gewährleistung einer guten oder zweckentsprechenden Bioverfügbarkeit molekulardispers in Arzneizubereitungen einzuarbeiten. Eine zu diesem Zweck ausreichend gute Löslichkeit besitzt die 5-Aminosalicylsäure nur in starken Alkalien. Bei diesen Bedingungen ist sie jedoch äußerst instabil. Innerhalb von wenigen Minuten verfärbt sich die 5-Aminosalicylsäure bei pH-Werten oberhalb von 10 bis 12 intensiv braun.

In WO 81/02671 werden 5-Aminosalicylsäure-Zubereitungen beschrieben. In dieser Druckschrift wird angegeben, daß ein Vorurteil gegen die orale Verabreichung von 5-Aminosalicylsäure besteht und daß ein oral verabreichbares Mittel hergestellt werden kann, wenn man 5-Aminosalicylsäure oder eines ihrer pharmazeutisch annehmbaren Salze oder einen Ester verwendet.

Der vorliegenden Erfindung liegt somit die Aufgabe zugrunde, Salze der 5-Aminosalicylsäure zur Verfügung zu stellen, die zu stabilen Arzneizubereitungen verarbeitet werden können, bei denen sich keine unvertretbaren Stabilitätsprobleme ergeben. Die 5-Aminosalicylsäure soll nach der Applikation der Arzneizubereitung in gelöster bzw. feinstverteilter Form am Wirkort aus der Arzneizubereitung freigesetzt werden. Die Salze sollen auf einfache Weise hergestellt werden können und die Anwendung der 5-Aminosalicylsäure in Form von Tabletten, Dragees, Kapseln etc. ermöglichen.

Gegenstand der Erfindung ist ein Salz aus 5-Aminosalicylsäure und Ammoniak, Ethanolamin, Diethanolamin, Triethanolamin, Tris-(hydroxymethyl)-aminomethan, N-Methylglukamin, Lysin, Arginin oder Cholin.

Gegenstand der Erfindung ist weiterhin eine Arzneimittelzubereitung, die ein Salz, wie oben angegeben, und gegebenenfalls einen Träger dafür enthält. Bevorzugt liegt die erfindungsgemäße Arzneimittelzubereitung in Form von Tabletten, Filmtabletten, Dragees, Kapseln oder Suppositorien vor. Es ist weiterhin bevorzugt, daß die Zubereitung magensaftresistent umhüllt ist.

Zur Herstellung der Salze kann man 5-Aminosalicylsäure mit einer konzentrierten wäßrigen Lösung von physiologisch und toxikologisch annehmbaren basischen Hilfsstoffen und/oder Puffergemischen, die als 1%ige wäßrige Lösung pH-Werte zwischen 8 und 12 ergeben, vermischen, mit einem geeigneten, mit Wasser mischbaren Lösungsmittel das gebildete Salz der 5-Aminosalicylsäure ausfällen, es von der wäßrigen Lösung abtrennen, trocknen und das erhaltene Salz in an sich bekannter Weise zu Tabletten, Filmtabletten, Dragees, Kapseln oder Suppositorien verarbeiten.

Bei dem Verfahren verwendet man als basische Hilfsstoffe oder Puffergemische solche, die in 1%iger wäßriger Lösung pH-Werte zwischen 8 und 12, vorzugsweise zwischen 9 und 11, aufweisen. Im Prinzip eignen sich alle anorganischen und organischen Verbindungen oder Puffergemische, die ausreichend stabil und ausreichend in Wasser löslich sind, die oben angegebenen pH-Werte in der wäßrigen Lösung ergeben und die physiologisch als Hilfsstoffe akzeptabel, d.h. innerlich ohne nennenswerte Wirkungen oder Nebenwirkungen einnehmbar, sind.

Darunter fallen Ammoniak, Ammoniumchlorid sowie ein Gemisch aus Ammoniak und Ammoniumchlorid.

Als basische Hilfsstoffe können auch organische Verbindungen, wie Ethanolamin (Colamin), Diethanolamin, Triethanolamin, Tris-(hydroxymethyl)-aminomethan (Tris-Puffer, THAM), N-Methylglukamin, Lysin, Arginin, Cholin etc., verwendet werden. Von den organischen Verbindungen sind Ethanolamin, Tris-(hydroxymethyl)-aminomethan, N-Methylglukamin, Lysin, Arginin und Cholin bevorzugt.

Günstige Voraussetzungen für eine Verwendung sind außerdem die Zulassung als Lebensmittelzusatz, als Arzneimittel-Hilfsstoff oder zumindest als unbedenklicher Hilfsstoff, ein kleines Molekulargewicht (damit beispielsweise die Tabletten nicht zu groß werden), eine leichte Beschaffbarkeit und ein nicht zu hoher Preis.

Bei dem Verfahren wird die 5-Aminosalicylsäure, wie oben ausgeführt, mit einer konzentrierten wäßrigen Lösung der oben beschriebenen basisch reagierenden Hilfsstoffe und/oder Puffergemische vermischt und in ausreichend stabiles und rasch lösliches Salz überführt. Die Hilfsstoffe und/oder Puffergemische werden hierzu, vorzugsweise unter Lichtschutz und Kühlung, in möglichst hoher Konzentration in Wasser gelöst. Bevorzugt werden mehr als ein Teil Hilfsstoff und/oder Puffergemisch in zwei Teilen Wasser gelöst. Diese Lösung wird mit der 5-Aminosalicylsäure, vorzugsweise unter Lichtschutz und Kühlung, vermischt. Die Vermischung erfolgt bevorzugt unter Rühren. Die 5-Aminosalicylsäure löst sich in der alkalisch reagierenden Lösung auf und bildet ein Salz. Zu der erhaltenen Lösung gibt man dann - vorzugsweise unter Rühren und Kühlen und ebenfalls unter Lichtausschluß - ein mit Wasser mischbares Lösungsmittel zur Ausfällung des entsprechenden Salzes der 5-Aminosalicylsäure. Als mit Wasser mischbare Lösungsmittel kann man beispielsweise Alkohole, wie Methanol, Ethanol oder Propanol, oder Ketone, wie Aceton, Methylethylketon, oder Tetrahydrofuran (THF) oder Dimethylformamid (DMF) bzw. Dimethylacetamid (DMA) oder diverse Glykolether etc. verwenden. Die Zugabe des Lösungsmittels erfolgt bevorzugt in einem Schuß innerhalb weniger Minuten. Anschließend wird unter 8°C abgekühlt und erforderlichenfalls Impfkristalle zugesetzt. Danach fällt das entsprechende Salz der 5-Aminosalicylsäure aus, welches möglichst schnell von der Mutterlauge abgetrennt und getrocknet wird.

Das erhaltene gut wasserlösliche Salz läßt sich in an sich bekannter Weise mit und ohne Verwendung von Arzneimittel-Trägerstoffen, wie oben ausgeführt, und gegebenenfalls unter Verwendung von üblichen Hilfsstoffen zu den verschiedensten Arzneizubereitungen verarbeiten. Beispielsweie kann man mit den Salzen, wie oben erwähnt, Tabletten, Filmtabletten, Dragees, Kapseln und Suppositorien herstellen. Erforderlichenfalls können auch diese Arzneizubereitungen magensaftresistent umhüllt werden.

Die Salze der 5-Aminosalicylsäure werden gegebenenfalls mit festen Arzneimittel-Trägerstoffen und üblichen Zusatzstoffen trocken vermischt und in an sich bekannter Weise brikettiert. Die Briketts werden anschließend in an sich bekannter Weise zu einem Granulat, beispielsweise zu einem Granulat mit einer mittleren Korngröße im Bereich von 0,6 mm bis 1,5 mm, vorzugsweise von 0,8 mm bis 1,0 mm, gebrochen. Dieses Granulat eignet sich besonders zur Herstellung von Tabletten, Drageekernen oder Kapseln mit Dosierungen von 200 mg bis 900 mg 5-Aminosalicylsäure pro Arzneiformeinheit. Werden feste Arzneiformen mit niedrigeren Dosierungen, beispielsweise mit 30 mg bis 175 mg pro Tablette oder Kapsel, hergestellt, dann sind die Granulate entsprechend feiner zu sieben, und zwar auf mittlere Korngrößen zwischen 0,15 mm bis 0,5 mm. Die Granulate können unter Einsatz üblicher Hilfsstoffe zu Tabletten, Dragees, Kapseln etc. verarbeitet werden. Direkt applizierbare Granulate, die anschließend nicht mehr tablettiert werden sollen, müssen größer als 1,5 mm gesiebt werden.

Beispiele für feste Trägerstoffe sind mikrokristalline Cellulose, Cellulosepulver, Carboxymethylcellulose, Methylcellulose, Stärke, Milchzucker, Saccharose, Dicalciumphosphat, Calciumsulfat etc.

Beispiele für flüssige Trägerstoffe sind wasserfreie Öle (Triglyceride), Paraffinöl, Polyethylenglykole mit den Molekulargewichten von 300 bis 600, Glycerin, Propylenglykol etc. Diese flüssigen Trägerstoffe eignen sich u.a. auch als Trägerstoffe zur Abfüllung von 5-Aminosalicylsäure in Weichgelatinekapseln.

Die Arzneizubereitung kann erforderlichenfalls, wenn es therapeutisch sinnvoll ist, in an sich bekannter Weise magensaftresistent umhüllt sein. Auf diese Weise wird sichergestellt, daß die Pufferwirksamkeit nicht durch den Einfluß des sauren Magensafts beeinträchtigt wird.

Beim Zusammentreffen mit physiologischen Flüssigkeiten in den Darmbereichen, die entweder in derartige Arzneizubereitungen eindiffundieren oder nach Auflösung der Umhüllung den Inhalt der betreffenden Arzneiform durchsetzen, lösen sich zunächst die Hilfsstoff- oder Puffergemische auf. Durch die hierbei entstehenden pH-Werte, die aus Stabilitätsgründen möglichst weit unter 12 liegen sollten, kann sich die 5-Aminosalicylsäure relativ rasch auflösen.

Die übliche Dosis von Tabletten und Suppositorien liegt heute bei 250 mg und 500 mg (für beide). Die Tagesdosis liegt bei 2 bis 4 g. Es ist möglich, daß sich diese Dosen durch die molekulardisperse Verteilung erniedrigen. Dies kann jedoch erst durch klinische Versuche bewiesen werden.

Die folgenden Beispiele erläutern die Erfindung, ohne sie zu beschränken.

### Beispiel 1

0,765 kg 5-Aminosalicylsäure werden in 1,462 kg einer 50%igen wäßrigen L-Lysinlösung durch gelinde Erwärmung und unter Lichtschutz gelöst. Nach Zusatz von ca. 1 l Ethanol und Abkühlung fällt ein gut wasserlösliches Salz aus, das abgenutscht und mit Aceton gewaschen wird. Nach dem Trocknen läßt sich das leicht rosa gefärbte Salz in praktisch jeder gewünschten Dosierung in die verschiedensten Arzneiformen einarbeiten.

### Beispiel 2

153,1 g 5-Aminosalicylsäure werden unter Lichtausschluß in 50- bis 70%igen wäßrigen Lösungen, welche beispielsweise entweder 195,2 g N-Methylglukamin oder 121,1 g Tris-(hydroxymethan)-methylamin oder 121,2 g Cholin enthalten, gelöst und anschließend im Vakuum getrocknet. Die so erhaltenen Salze werden mit Aceton oder einem Alkohol gewaschen und nochmals getrocknet. Sie sind sehr gut wasserlöslich und so stabil, daß sie sich ohne Schwierigkeiten in Arzneiformen einarbeiten lassen.

## Patentansprüche

1. Salz aus 5-Aminosalicylsäure und Ammoniak, Ethanolamin, Diethanolamin, Triethanolamin, Tris-(hydroxymethyl)-aminomethan, N-Methylglukamin, Lysin, Arginin oder Cholin.

2. Arzneimittelzubereitung, dadurch **gekennzeichnet,** daß sie ein Salz nach Anspruch 1 und gegebenenfalls einen Träger dafür enthält.

3. Zubereitung nach Anspruch 2, dadurch **gekennzeichnet,** daß sie als Tabletten-, Filmtabletten-, Dragee-, Kapsel- oder Suppositorien-Zubereitung vorliegt.

4. Zubereitung nach einem der Ansprüche 2 oder 3, dadurch **gekennzeichnet,** daß die Zubereitung magensaftresistent umhüllt ist.

## Claims

1. A salt of 5-aminosalicylic acid and ammonia, ethanolamine, diethanolamine, triethanolamine, tris-(hydroxymethyl)-aminomethane, N-methyl glucamine, lysine, arginine or choline.

2. A medicinal preparation, characterized in that it contains a salt according to claim 1 and optionally a carrier therefor.

3. A preparation as claimed in claim 2, characterized in that it is formulated as tablets, film tablets, dragees, capsules or suppositories.

4. A preparation as claimed in claim 2 or 3, characterized in that it has a coating resistant to gastric juices.

## Revendications

1. Sel d'acide 5-aminosalicylique et d'ammoniac, d'éthanolamine, de diéthanolamine, de triéthanolamine, de tris-(hydroxyméthyl)aminométhane, de N-méthylgucamine, de lysine, d'arginine ou de choline.

2. Préparation pharmaccutique, caractérisée en ce qu'elle contient un sel selon la revendication 1 et éventuellement un support pour ce sel.

3. Préparation selon la revendication 2, caractérisée en ce qu'elle est sous foie de comprimés, de comprimés dragéifiés, de dragées, de gélules ou de suppositoires.

4. Préparation selon la revendication 2 ou 3, caractérisée en ce que la préparation est enrobée dans un revêtement résistant au suc gastrique.
